# FASCICULE DE BREVET EUROPEEN

(11) **EP 3 383 526 B1**
(45) Date de publication et mention de la délivrance du brevet: **08.01.2020**
(21) Numéro de dépôt: 16802048.5
(22) Date de dépôt: 28.11.2016
(51) Int. Cl.: B01F 17/50, B01F 17/00, A61K 8/27, A61K 8/29, A61K 8/72, A61Q 17/04, A61K 8/06

(54) **EMULSIONS ANTI-UV STABILISEES AVEC DE LA LIGNINE ET DES NANOPARTICULES**
MIT LIGNIN UND NANOPARTIKELN STABILISIERTE ANTI-UV-EMULSIONEN
ANTI-UV EMULSIONS STABILISED WITH LIGNIN AND NANOPARTICLES

(30) Priorité: 30.11.2015 FR 1561575
(43) Date de publication de la demande: 10.10.2018
(73) Titulaire: Centre National de la Recherche Scientifique (CNRS), 75016 Paris (FR); Université d'Orléans, 45067 Orléans (FR)
(72) Inventeur: HAMBARDZUMYAN, Arayik, 45000 Orléans (FR)
(74) Mandataire: Lavoix
(86) Numéro de dépôt international: PCT/EP2016/078995
(87) Numéro de publication internationale: WO 2017/093184

(56) Documents cités:
- EP-A1- 0 518 773
- WO-A1-2012/156652
- DD-A1- 249 859
- JP-A- H0 433 986
- STEPHANIE LAM ET AL: "Pickering stabilization of foams and emulsions with particles of biological origin", CURRENT OPINION IN COLLOID AND INTERFACE SCIENCE, vol. 19, no. 5, 1 octobre 2014 (2014-10-01), pages 490-500, XP055291601, GB ISSN: 1359-0294, DOI: 10.1016/j.cocis.2014.07.003

## Description

La présente invention se rapporte à des émulsions, et plus précisément à des émulsions de type « huile-dans-eau » comprenant une couche d'adsorption entre les deux phases présentant des propriétés anti-oxydantes et anti-rayonnements ultra-violets.

### INTRODUCTION

Les émulsions sont largement produites et utilisées dans l'industrie soit comme matériaux à consommer, soit à appliquer sur des surfaces en tant que vecteurs d'agents non solubles dans l'eau. On retrouve des émulsions en pharmacie, en cosmétique (laits, crèmes, pommades), en cuisine (sauces, crèmes), en peinture, dans l'industrie routière, en agrochimie, en détergence, dans le laminage, la sidérurgie et dans la fabrication de dépôts divers (imprimerie, adhésifs ...).

En cosmétique et en pharmacie, les émulsions constituent un moyen efficace d'obtenir une combinaison harmonieuse d'ingrédients de nature et de propriétés différentes, notamment d'ingrédients lipophiles et hydrophiles, en une présentation homogène et facile d'emploi.

Une émulsion peut être définie comme un mélange intime de deux substances liquides, non miscibles, consistants en une phase aqueuse et une phase huileuse ou grasse. Par action mécanique et/ou chimique, la création d'une émulsion permet de mélanger ces deux phases, l'une des phases étant dispersée dans la seconde sous forme de petites gouttelettes.

On distingue les émulsions de type « huile dans l'eau » et « eau dans huile ». Ainsi, une émulsion huile-dans-eau (H/E ou O/W pour oil in water) est composée d'une phase huileuse dispersée dans une phase aqueuse. Il s'agit d'une émulsion « directe ».

A l'inverse, une émulsion eau-dans-huile (E/H ou W/O pour water in oil) est composée d'une phase aqueuse dispersée dans une phase huileuse. Une émulsion E/H est plus grasse au toucher, car le toucher correspond majoritairement à la nature de la phase externe. Une telle émulsion est dite « inverse ».

D'un point de vue thermodynamique, les émulsions sont par nature instables, notamment au cours du temps. Le mélange reste stable grâce à la présence d'un ingrédient appelé émulsifiant ou émulsionnant.

Les émulsifiants sont le plus souvent des tensioactifs ou des agents de surface. Les émulsifiants peuvent être de nature ionique ou non-ionique. Les molécules utilisées peuvent être d'origine naturelle ou synthétique.

Certains émulsifiants présentent des avantages spécifiques, additionnels à leurs propriétés de stabilisation. Ainsi, certains émulsifiants assurent la protection des principes actifs contre l'oxydation, contre les rayons ultraviolets, et/ou leur conservation au sein des émulsions. Ces émulsifiants qui permettent de protéger les principes actifs lipophiles, contenus dans la phase grasse, sont activement recherchés. En effet, la stabilité oxydative des molécules actives dans les émulsions est un point particulièrement important dans l'industrie cosmétique.

Les radicaux libres tels que les hydroxyles, peroxyles et superoxyles sont des déclencheurs d'oxydation des molécules actives. Ces radicaux sont générés soit dans la phase aqueuse, soit à l'interface huile/eau des émulsions, sous l'effet de différents paramètres extérieurs tels que la lumière et l'oxygène. Ces radicaux libres peuvent passer à travers la couche d'adsorption, et ainsi initier des réactions oxydatives des produits encapsulés dans la phase lipidique. Ce phénomène d'oxydation est accéléré en présence de rayons UV.

Parmi les produits sensibles à l'oxydation, on peut citer les huiles végétales, qui souvent constituent une bonne portion de la phase grasse des compositions cosmétiques. Lorsque les huiles sont oxydées, elles présentent une odeur rance et un changement de couleur. On peut citer également les vitamines, qui peuvent être dégradées suite à une exposition plus ou moins longue aux rayons ultra-violets.

Ainsi, les émulsions contenant des principes actifs lipophiles fragiles, notamment ceux étant dégradés/oxydés lorsque exposés trop longtemps à la lumière, doivent être maintenues dans des contenants opaques ou en verre fumé, et de préférence dans l'obscurité totale, afin de préserver leurs qualités au cours du temps.

Ceci génère un réel coût pour le fabriquant qui doit utiliser des contenants adaptés, et est un désavantage pour l'utilisateur, qui doit maintenir l'émulsion dans des conditions appropriées pour conserver les qualités des principes actifs et des huiles présents dans ladite émulsion.

Afin de s'affranchir de cette nécessité d'utiliser un contenant approprié, des composés présentant des propriétés de protection contre l'oxydation et les dommages causés par les rayons ultraviolets (UV), ont été identifiés et intégrés dans les couches d'adsorption se trouvant à l'interface des phases aqueuse et grasse.

En effet, la structure de la couche d'adsorption peut faire diminuer de façon très significative la survenue des réactions d'oxydations, en limitant les interactions des produits encapsulés dans la phase lipidique avec les radicaux libres et l'oxygène présents dans la phase aqueuse, et/ou en absorbant ou dispersant les rayons ultraviolets.

Ainsi, cette couche d'adsorption peut être constituée de composés possédant des capacités anti-oxydantes et de filtres UV propres, et/ou comprendre des composés présentant une capacité à potentialiser les effets d'autres composés actifs, protégeant ainsi les composés lipophiles contre l'oxydation et les dommages causés par les rayons ultraviolets.

Parmi les composés connus pour leurs propriétés de filtres UV, on peut citer les nanoparticules d'oxyde métallique, classiquement utilisées dans les compositions cosmétiques à visée de protection solaire. Les nanoparticules d'oxyde métallique et leur utilisation en tant qu'agents photoprotecteurs sont connus et décrits par exemple dans les demandes de brevet EP 518772 et EP 518773. Ces nanoparticules permettent de protéger les matériaux auxquels elles sont associées des rayonnements visibles et des rayonnements ultraviolets, en absorbant les rayonnements dans la gamme de longueurs d'onde allant de 250 nm à 400 nm.

On peut également citer la lignine, un polymère naturel capable d'absorber les rayons ultraviolets. Il a récemment été montré que la lignine peut avantageusement être intégrée à des films absorbeurs d'UV, comprenant également des nanocristaux de cellulose (WO 2012/156652). La lignine possède également des propriétés anti-oxydantes (Pouteau *et al.,* 2003). Par ailleurs, la lignine agit comme un émulsifiant permettant de stabiliser des émulsions (Rojas *et al.,* 2007). et Lam et al. en "Current Opinion in Colloid & Interface Science", 19 (2014), p. 490-500.

JP 04033986 décrit un film lignocellulosique comme absorbeur UV. La demande de brevet US 2002/0182155 décrit des compositions comprenant une combinaison de charges particulaires (argile par exemple) et de lignine, qui permet de potentialiser l'efficacité de composés capables d'absorber les radiations UV, tels que des nanoparticules de dioxyde de titane (TiO₂). D'importantes quantités de dioxyde de titane et/ou de lignine sont utilisées dans ces compositions pour obtenir un facteur de protection solaire ('SPF' pour Sun Protection Factor) satisfaisant.

Le problème que se propose de résoudre la présente invention consiste à réduire, dans les émulsions, les quantités utilisées de lignine et surtout de nanoparticules d'oxyde métallique, tout en maintenant une protection suffisante contre les effets néfastes des rayons UV et les radicaux libres.

En effet, différentes études ont mis en évidence le fait que les nanoparticules de dioxyde de titane et de monoxyde de zinc, utilisées en cosmétique et notamment dans les crèmes solaires, sont potentiellement toxiques. A l'heure actuelle, les potentiels effets néfastes de ces composés sont activement recherchés par les scientifiques (Shi *et al.,* 2013) et (Rapport de l'AFSSAPS relatif aux nanomatériaux dans les produits cosmétiques, adopté le 15/03/2011).

Néanmoins, il n'existe pas de composés présentant des capacités similaires d'absorbance des rayons UV ; ainsi, la recherche s'oriente vers la détermination de conditions particulières qui permettent de diminuer les doses utilisées de nanoparticules d'oxyde métallique, tout en maintenant une absorbance efficace des rayons UV.

### RESUME DE L'INVENTION

La présente invention concerne une émulsion de type « huile-dans-eau », comprenant une couche d'adsorption permettant à la fois la stabilité de l'émulsion, et la protection des principes actifs lipophiles contre les rayonnements UV et contre les agents d'oxydation.

Ladite couche d'adsorption est constituée d'une matrice de lignine, comprenant des nanoparticules d'oxyde métallique, en des proportions particulières, permettant d'optimiser les propriétés anti-oxydantes et anti-UV de cette couche d'adsorption tout en minimisant les quantités utilisées des constituants de cette couche d'adsorption.

La présente invention concerne une émulsion de type « huile-dans-eau » comprenant une phase aqueuse, une phase grasse, et une couche d'adsorption à l'interface des deux phases, ladite couche d'adsorption étant constituée d'une matrice de lignine dans laquelle sont incorporées des nanoparticules d'oxyde métallique, caractérisée en ce que la lignine est présente en une quantité inférieure à 2% en poids, et que les nanoparticules d'oxyde métallique sont présentes en une quantité inférieure à 1.5% en poids, par rapport au poids total de l'émulsion.

La présente invention concerne l'amélioration des propriétés de cette couche d'adsorption composée de lignine, par incorporation dans la matrice de lignine de nanoparticules d'oxyde métallique, cette incorporation permettant notamment d'améliorer :
- les propriétés rhéologiques de ladite couche d'adsorption ; et
- la capacité des nanoparticules d'oxyde métallique à absorber les rayons UV, et ainsi la possibilité de diminuer leur quantité.

### FIGURES

**Figure 1**. Absorbance en UV de la molécule de lignine seule. Les différentes longueurs d'onde sont présentées sur l'axe des abscisses, l'intensité de la capacité d'absorbance de la molécule de lignine est indiquée sur l'axe des ordonnées.
**Figure 2**. Absorbance en UV de la molécule de lignine en solution aqueuse, seule ou en présence de différentes nanoparticules d'oxyde métallique : TiO₂ (Eusolex T2000, Eusolex TS, organosolve) et ZnO.
**Figure 3**. Cinétique d'adsorption de la lignine à l'interface phase grasse / phase aqueuse : effets de la présence de nanoparticules d'oxyde métallique sur la tension de surface (mN/m), en fonction du temps, représenté sur l'axe des adscisses.
**Figure 4****.** Facteur de protection solaire (SPF) et facteur de protection contre les UVA (FP-UVA) obtenus en fonction de la concentration de nanoparticules de TiO₂ (représentée sur l'axe des abscisses), en présence de 1.2% de lignine.

### DESCRIPTION DETAILLEE

La présente invention concerne une émulsion de type « huile-dans-eau » comprenant une phase aqueuse, une phase grasse, et une couche d'adsorption à l'interface des deux phases, ladite couche d'adsorption étant constituée d'une matrice de lignine dans laquelle sont incorporées des nanoparticules d'oxyde métallique, caractérisée en ce que la lignine est présente en une quantité de 0.5% à 2% en poids, et que lesdites nanoparticules sont présentes en une quantité de 0.5% à 1.5% en poids, par rapport au poids total de l'émulsion.

Il est entendu que les valeurs des bornes inférieures et supérieures des gammes indiquées dans la présente demande sont comprises dans l'intervalle ainsi désigné.

Comme indiqué précédemment, une émulsion est une dispersion, sous forme de gouttelettes, de deux liquides non miscibles entres eux. Elle est constituée d'une phase aqueuse et d'une phase grasse. Une émulsion de type huile-dans-eau est caractérisée en ce que la phase grasse est dispersée, sous forme de gouttelettes, dans la phase aqueuse.

Au sens de l'invention, on entend par phase aqueuse la phase qui comprend de l'eau et/ou au moins un solvant hydrosoluble, c'est à dire un composé liquide à température ambiante et miscible à l'eau. La phase aqueuse peut également comprendre tout composé hydrosoluble ou hydrodispersible compatible avec une phase aqueuse tels que des gélifiants, des polymères filmogènes, des épaississants, des tensio-actifs et leurs mélanges.

Au sens de l'invention, on entend par phase grasse la phase qui inclut tout corps gras liquide, généralement une huile ou un hydrocarbure. Cette phase peut également être désignée par phase huileuse, organique ou lipophile, ces adjectifs étant utilisés indifféremment et désignant la même phase dans la présente demande.

Au sens de l'invention, le terme 'couche d'adsorption' désigne la couche se formant à l'interface phase aqueuse/phase grasse dans l'émulsion, et permettant de maintenir la stabilité de la dispersion des gouttelettes d'huile dans la phase aqueuse.

On entend par «filtres ultraviolets» ou « filtres UV », les substances qui absorbent, réfléchissent ou dispersent ces rayonnements. Au sens de la présente invention, ces substances sont destinées à protéger les produits lipophiles contenus dans la phase grasse des émulsions.

Au sens de l'invention, l'expression « matrice de lignine » désigne le réseau tridimensionnel constitué de lignine qui prend forme à l'interface des phases aqueuse et grasse, et constitue la « base » de la couche d'adsorption autour des microgouttes de la phase huileuse.

### La lignine

La lignine est un des principaux composants du bois, avec la cellulose et l'hémicellulose. Ses principales fonctions sont d'apporter de la rigidité, une imperméabilité à l'eau et une grande résistance à la décomposition. Toutes les plantes vasculaires, ligneuses et herbacées, fabriquent de la lignine.

La lignine est le deuxième biopolymère renouvelable le plus abondant sur la Terre, après la cellulose. Ce polymère est constitué d'au moins trois types de monomères différents, les monolignols suivants :
- alcool coumarylique, appelé unité H (hydroxyphényle), sans groupe méthoxy ;
- alcool coniférylique, appelé unité G (guaïacyle), à un groupe méthoxy ;
- alcool sinapylique, appelé unité S (syringyle), à deux groupes méthoxy..

La fraction de chaque monomère varie de façon importante en fonction de la lignée végétale (gymnosperme, angiosperme monocotylédone, angiosperme dicotylédone) ; de l'espèce ; de l'organe ; du tissu ; et d'une manière générale de l'environnement physico-chimique dans lequel le végétal croît.

Il n'y a donc pas une définition unique et précise de la lignine, du fait de sa grande variabilité et cela au sein même d'une espèce donnée.

La lignine est un polymère qui adopte une structure de réseau en trois dimensions, aussi appelé 'grille' ou 'matrice', ledit réseau permettant des interconnections entre les couches d'adsorption présentes autour de chaque gouttelette de phase grasse. Cette matrice présente des propriétés mécaniques tout à fait intéressantes, permettant à la fois une très bonne stabilisation de l'émulsion et une bonne résistance mécanique de la couche d'adsorption.

La lignine utilisée dans l'émulsion selon l'invention est préférentiellement une lignine d'origine naturelle.

Les lignines naturelles sont préférentiellement extraites de plantes vasculaires ligneuses ou herbacées en réalisant une acidolyse dans un solvant organique. Par exemple, le mode d'extraction dans un milieu dioxane/eau, en présence d'acide chlorhydrique, permet d'obtenir des préparations de lignines relativement peu modifiées et pauvres en sucres associés (Monties B, 1988).

Selon un aspect particulier de l'invention, la lignine utilisée dans l'émulsion selon l'invention est issue d'un procédé d'extraction de cellulose à partir de plantes.

Selon un autre aspect de l'invention, la lignine utilisée dans l'émulsion selon l'invention est d'origine synthétique.

La lignine d'origine synthétique, aussi appelée 'DHP' pour 'dehydropolymere', peut être synthétisée *in vitro* en deux étapes :
(i) synthèse de l'alcool coniférylique ou autre monolignol et
(ii) polymérisation des monolignols en présence d'eau oxygénée et de peroxydase.

Il est entendu que la lignine utilisée dans l'émulsion selon l'invention peut être purifiée ou non, et peut avoir été modifiée chimiquement.

L'émulsion selon l'invention comprend donc une couche d'adsorption constituée d'une matrice de lignine dans laquelle sont incorporées des nanoparticules d'oxyde métallique, en des quantités spécifiques.

### Nanoparticules d'oxyde métallique

Les nanoparticules d'oxyde métallique sont ici utilisées en tant que filtres UV, destinés à absorber, réfléchir ou disperser les rayonnements UV.

Le métal dudit oxyde métallique est avantageusement choisi dans le groupe constitué par le titane, le zinc, le cérium, le zirconium, le fer, le cuivre, et leurs mélanges, de préférence dans le groupe constitué par le titane, le cérium, le zinc, et leurs mélanges. De manière préférée le métal est le titane.

Selon un mode de réalisation, ledit oxyde métallique est choisi dans le groupe constitué par le dioxyde de titane (TiO₂), le monoxyde de zinc (ZnO), les oxydes de cérium (Ce₂O₃ et CeO₂), le dioxyde de zirconium (ZrO₂) et les oxydes de cuivre (CuO et Cu₂O).

Selon un aspect préféré de l'invention, les nanoparticules d'oxyde métallique sont des nanoparticules de monoxyde de zinc (ZnO) de numéro CAS 1314-13-2, ou de dioxyde de titane (TiO2) de numéro CAS 13463-67-7, ces deux types de nanoparticules pouvant être incorporées à des compositions cosmétiques d'un point de vue règlementaire.

On préfère tout particulièrement les nanoparticules de dioxyde de titane.

Il est entendu que ces nanoparticules peuvent être utilisées sous forme de produits commerciaux qui peuvent comprendre jusqu'à 30% d'autres agents ou excipients. Avantageusement, on choisira des produits comprenant au moins 70% de nanoparticules d'oxyde métallique.

A titre informatif, différents composés commerciaux comprenant des nanoparticules pouvant être utilisées dans les émulsions de l'invention sont répertoriés dans les tableaux ci-dessous :

**Tableau 1**

| **Nom commercial** | **Nom INCI** | **% de nanoparticules d'oxyde métallique** |
|---|---|---|
| Oxyde de titane standard | Titanium dioxide | 99-100% |
| Eusolex T-Avo® | Titanium dioxide, Silica | 79,6% |
| Eusolex T-2000® | Titanium dioxide, Alumina, Simethicone | 80,3% |
| Eusolex T-Eco® | Titanium dioxide, Alumina, simethicone | 79,6% |
| Eusolex T® | Titanium dioxide, Simethicone | 77,5% |
| Eusolex T-S® | Titanium dioxide, Alumina, Stearic acid | 73-79% |

**Tableau 2**

| **Noms commerciaux** | **Noms INCI** | **% de nanoparticules d'oxyde métallique** |
|---|---|---|
| Zinc oxide Neutral® | Zinc oxide | Min 95% |
| Zinc oxide NDM® | Zinc oxide, Dimethicone | Min 92% |
| Z-Cote® | Zinc oxide | 99-100% |
| Z-Cote Max® | Zinc oxide, Diphenyl Capryl methicone | 96-99% |
| Tego-Sun Z500® | Zinc oxide | >99,5% |
| Tego-Sun Z800® | Zinc oxide, Trimetoxycaprylylsilane | >94% |
| Nanox 200® | Zinc oxide | 99% |

De manière tout à fait préférée, les nanoparticules de dioxyde de titane utilisées sont choisies parmi celles de la marque Eusolex T-2000® et Eusolex T-S®, ou un mélange des deux.

Selon un autre aspect de l'invention, un mélange d'au moins deux types de nanoparticules d'oxyde métallique différentes est utilisé. Il pourra notamment être fait usage d'un mélange de nanoparticules de monoxyde de zinc et de dioxyde de titane.

Par nanoparticules, on entend des nanoparticules possédant un diamètre moyen compris entre 2 nm et 100 nm, ou entre 2 nm et 50 nm, avantageusement entre 4 nm et 30 nm, de préférence entre 6 nm et 20 nm, de préférence encore entre 8 nm et 10 nm.

Des nanoparticules d'oxyde métallique de diamètre moyen inférieur à 2 nm peuvent être envisagées. Cependant, lorsque le diamètre moyen est inférieur à la taille des pores du derme, lesdites nanoparticules peuvent traverser la peau, ce qui ne représente pas un mode de réalisation préféré de la présente invention.

Les nanoparticules d'oxyde métallique sont incorporées à la matrice créée par le polymère de la lignine. Lesdites nanoparticules sont ainsi « intégrées » ou « emprisonnées » dans ce réseau de polymère. Toute technique consistant à mélanger et homogénéiser les deux composés est adaptée à la mise en œuvre de l'invention. Il est préférable d'obtenir une bonne uniformité de la répartition des particules dans la matrice de lignine.

Selon une première mise en œuvre de l'invention, les nanoparticules d'oxyde métallique sont liées à la matrice de lignine par des liaisons non-covalentes. Lesdites nanoparticules sont incorporées dans la matrice de lignine et restent en place grâce à des liaisons non-covalentes existant entre ces molécules. On désigne ainsi des liaisons permettant l'assemblage de molécules, telles que les liaisons hydrogène, la coordination métallique, les forces hydrophobes, les forces de van der Waals, l'empilements (π-π), l'électrostatique et les entre-actions électromagnétiques.

Selon un aspect préféré de l'invention, les nanoparticules d'oxyde métallique sont incorporées à la matrice de lignine par sonication.

La technique de sonication est basée sur l'utilisation d'appareils 'sonicateurs' produisant des ondes dans les ultra-sons, qui agitent les molécules et permettent ainsi leur homogénéisation.

Selon une seconde mise en œuvre de l'invention, les nanoparticules d'oxyde métallique sont liées à la matrice de lignine par des liaisons covalentes. Une liaison covalente est une liaison chimique dans laquelle deux atomes partagent deux électrons d'une de leurs couches externes afin de former un doublet d'électrons liant les deux atomes.

Une interaction covalente entre ces molécules peut être obtenue notamment lorsqu'une liaison chimique s'établit entre l'orbital vide vacante de l'oxyde métallique, et le doublet électronique de l'oxygène, atome très abondant dans la molécule de lignine.

L'incorporation de ces nanoparticules au sein de la matrice lignine permet d'optimiser leur capacité de filtres UV, et ainsi permet de diminuer la quantité de nanoparticules utilisées tout en conservant des propriétés de protection contre les rayons UV satisfaisantes.

### Quantités et rapports considérés selon l'invention

La présente invention est relative à une émulsion comprenant une couche d'adsorption constituée d'une matrice de lignine dans laquelle sont incorporées des nanoparticules d'oxyde métallique, caractérisée en ce que la lignine est présente en une quantité de 0.5% à 2% en poids, et que lesdites nanoparticules sont en une quantité de 0.5% à 1.5% en poids, par rapport au poids total de l'émulsion.

Comme précédemment présenté, il est en effet essentiel de réduire dans les émulsions la quantité de ces composés « annexes » qui ne sont pas des composés actifs, tout en conservant leurs propriétés stabilisantes de l'émulsion et protectrices des composés actifs contre les rayons UV et les agents oxydants.

Une faible quantité de nanoparticules d'oxyde métallique dans le système émulsionné est préférable pour toutes les raisons cités précédemment ; de plus, à des concentrations élevées, il a été observé que le pouvoir absorbeur des nanoparticules d'oxyde métallique est atténué.

Selon un premier aspect, la lignine est présente dans l' une quantité de 0.5% à 2% en poids par rapport au poids total de l'émulsion. La lignine peut notamment être présente en une quantité de 2, 1.9, 1.8, 1.7, 1.6, 1.5, 1.4, 1.3, 1.2, 1.1, 1, 0.9, 0.8, 0.7, 0.6, ou 0.5% en poids, par rapport au poids total de l'émulsion.

Selon un second aspect, les nanoparticules d'oxyde métallique sont présentes dans l'émulsion en une quantité de 0.5% à 1.5% par rapport au poids total de l'émulsion. Lesdites nanoparticules peuvent notamment être présentes en une quantité de 1.5, 1.4, 1.3, 1.2, 1.1, 1, 0.9, 0.8, 0.7, 0.6, ou 0.5% en poids, par rapport au poids total de l'émulsion.

Selon un aspect préféré de l'invention, dans ces émulsions, le ratio en poids nanoparticules d'oxyde métallique / lignine est inférieur à 1.

Plus particulièrement, dans ces émulsions, le ratio en poids nanoparticules d'oxyde métallique / lignine est inférieur à 1, inférieur à 0.9, inférieur à 0.8, inférieur à 0.7, inférieur à 0.6, inférieur à 0.5, inférieur 0.4, inférieur à 0.3 ou inférieur à 0.2.

Selon un aspect préféré de l'invention, les nanoparticules d'oxyde métallique sont présentes en un ratio en poids nanoparticules / lignine compris entre 0.1 et 0.8, et plus préférentiellement sont présentes en un ratio en poids nanoparticules / lignine d'environ 0.5.

Selon un aspect particulier de l'invention, l'émulsion de type « huile-dans-eau » consiste en une phase aqueuse, une phase grasse, et une couche d'adsorption à l'interface des deux phases, ladite couche d'adsorption étant constituée d'une matrice de lignine dans laquelle sont incorporées des nanoparticules d'oxyde métallique, l'émulsion ne comprenant pas d'épaississant à base de particules.

On entend notamment par «épaississant à base de particules » des épaississants tels que des argiles smectites, qui comprennent la montmorillonite, la bentonite, la bidelite, l'hectorite, la saponite, la stevensite etc.

Il est entendu que, selon cette mise en œuvre, les phases aqueuse et grasse sont constituées de différents composés lipophiles (dans la phase grasse) et hydrophiles (dans la phase aqueuse) mais ne comprennent pas d'épaississant particulaire.

Selon un aspect particulier de l'invention, la phase grasse peut représenter au moins 50% en poids de l'émulsion, par rapport au poids total de l'émulsion. On désigne une telle émulsion une « émulsion concentrée ».

Notamment, l'émulsion peut comprendre au moins 50%, 55%, 60%, 65%, 70%, 75%, 80%, ou 85% en poids de phase grasse, relativement au poids total de l'émulsion.

Selon un aspect préféré de l'invention, la phase grasse représente entre 50 et 70% en poids de l'émulsion.

La phase grasse de l'émulsion selon l'invention comprend au moins un corps gras choisi parmi les corps gras liquides à température ambiante (20-25°C) tels que les huiles, volatiles ou non, d'origine végétale, minérale ou synthétique, et leurs mélanges. La phase grasse peut comprendre aussi tout additif habituel, liposoluble ou lipodispersible.

Pour une application dans le domaine cosmétique, ces huiles sont choisies parmi des huiles physiologiquement acceptables.

Comme huiles utilisables dans la composition de l'invention, on peut citer par exemple :
- les huiles hydrocarbonées d'origine animale;
- les huiles hydrocarbonées d'origine végétale;
- les esters et les éthers de synthèse, notamment d'acides gras;
- les hydrocarbures linéaires ou ramifiés, d'origine minérale ou synthétique, tels que les huiles de paraffine, volatiles ou non, et leurs dérivés;
- les huiles fluorées partiellement hydrocarbonées et/ou siliconées;
- les huiles de silicone;
- et leurs mélanges.

Parmi les hydrocarbures de formule brute CₙHₘ, avec n et m étant deux chiffres entiers, les hydrocarbures linéaires, aromatiques et cycliques peuvent être utilisés, et ce quel que soit leur point d'ébullition.

Les huiles essentielles, notamment extraites à partir de plantes, sont des hydrocarbures préférés pour la mise en œuvre de l'invention.

On citera plus particulièrement comme hydrocarbure pouvant être présent dans la phase grasse de l'invention le cyclopentane (C₅H₁₀), l'hexane (C₆H₁₄), le méthylcyclohexane (C₇H₁₄), l'heptane (C₇H₁₆), le décane (C₁₀H₂₂), le dodécane (C₁₂H₂₆) et le toluène (C₇H₈).

La phase grasse dans l'émulsion selon l'invention pourra être composée d'une seule huile, en particulier d'un seul hydrocarbure, ou pourra également être constituée d'un mélange de deux, trois voire quatre huiles différentes.

Selon un aspect de l'invention, la phase grasse comprend un solvant organique choisi parmi le cyclopentane, l'hexane, le méthylcyclohexane, l'heptane, le décane, le dodécane et le toluène.

Selon un autre aspect de l'invention, la phase grasse est principalement constituée, ou totalement constituée, d'un solvant organique choisi parmi le cyclopentane, l'hexane, le méthylcyclohexane, l'heptane, le décane, le dodécane et le toluène.

La phase aqueuse de l'émulsion selon l'invention comprend principalement de l'eau et éventuellement un ou plusieurs composés miscibles à l'eau. La phase aqueuse peut comprendre aussi des espèces ioniques, des régulateurs de pH, et tous les principes actifs, conservateurs et colorants qui sont hydrosolubles ou hydrodispersibles.

### EXEMPLES

Les produits utilisés dans cette étude sont de marque SIGMA-ALDRICH et sont utilisés sans purification complémentaire :
Décane > 95%
Lignine: Lignin, alkali low sulfonate content 471003 Aldrich
Nanoparticules de ZnO
Nanoparticules de TiO2 :
   a) TiO2 (organosolve W877).
   b) TiO2 (Eusolex T-S®)
   c) TiO2 (Eusolex T-2000®)

### Exemple 1. Préparation de solutions aqueuses de lignine et de nanoparticules d'oxyde métallique

Afin de vérifier la miscibilité de ces nanoparticules dans une matrice de polymère de la lignine, nous les avons mélangés avec la solution aqueuse de lignine (0.1g lignine solubilisées dans 3.5ml d'eau) par sonication selon les conditions suivantes : pendant 7 minutes, 1 seconde 'up', 1 seconde 'off', amplitude 30%, à T°=25°C, avec un sonicateur Vibra cell 75115 de marque BIOBLOCK SCIENTIFIC.

Après évaporation complète de la phase aqueuse, des images en microscopie électronique à balayage (MEB) ont permis d'observer que les nanoparticules de ZnO et de TiO2 ont une grande affinité pour la lignine et qu'elles « pénètrent » parfaitement dans la matrice constituée par ce polymère naturel.

### Exemple 2. Préparation d'émulsions décane/eau stabilisées avec de la lignine et des nonaparticules d'oxyde métallique

Les émulsions sont préparées par une agitation des phases huile et eau en présence de la lignine par sonication comme décrit ci-dessus.
Des émulsions concentrées composées de 6.5 décane /3.5 eau (v/v) sont stabilisées avec 100 mg de lignine sans nanoparticule (1), ou avec de la lignine additionnée de 50mg ZnO (2), 50mg TiO2 W877 Organoslove (3), 50 mg TiO2 (Eusolex TS) (4) ou 50 mg TiO2 (Eusolex T2000) (5).

Après complète évaporation des phases aqueuse et organique de ces émulsions, la couche d'adsorption de chacune de ces émulsions a été prise en photo. Les images en microscopie électronique à balayage des couches d'adsorption permettent d'observer que la couche d'adsorption présente une structure poreuse assez régulière.

### Exemple 3. Absorbance en UV des solutions aqueuse de lignine en combinaison avec différents types de nanoparticules d'oxyde métallique

Les solutions aqueuse de la lignine d'une concentration 30g/l, avec et sans nanoparticules de ZnO et TiO2, sont préparées. Toutes les solutions sont mélangées par sonication pendant 7 minutes, avec des pulsations de 0.1 seconde 'on', 0.1 seconde 'off'.

**Tableau 3**

| Composé | Lignine (g/l) | ZnO (g/l) | TiO2 (W877) (g/l) | TiO2 (Euso TS) (g/l) | TiO2 (Euso T2000) (g/l) |
|---|---|---|---|---|---|
| Ech-1 (lignine) | 0.055 | | | | |
| Ech-2 (lignine+ZnO) | 0.055 | 0.025 | | | |
| Ech-3 (lignine+TiO2, W877) | 0.055 | | 0.025 | | |
| Ech-4 (lignine+TiO2, Eusolex TS) | 0.055 | | | 0.025 | |
| Ech-5 (lignine+TiO2, Eus T2000) | 0.055 | | | | 0.025 |

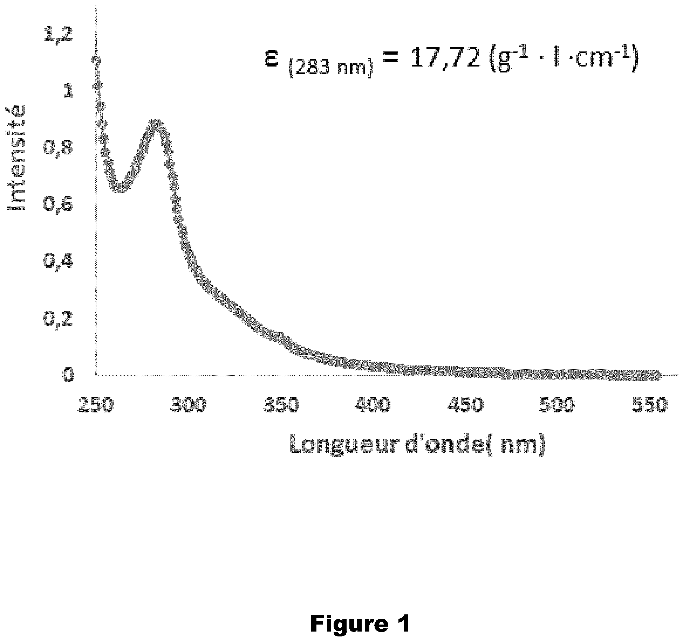

Comme cela est montré en figure 1, la lignine seule absorbe de manière significative les rayons à la longueur d'ondes de 280 nm.

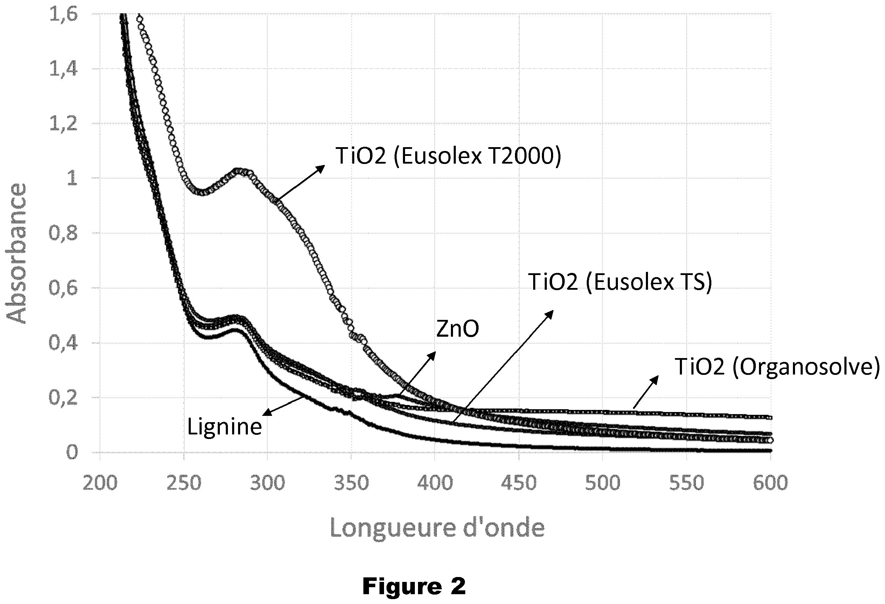

La figure 2 montre qu'en présence de nanoparticules de TiO2 (Eusolex T2000) on observe une augmentation importante en intensité d'absorbance de la combinaison lignine + nanoparticules.

### Exemple 4. Cinétique d'adsorption de la lignine en combinaison avec différents types de nanoparticules d'oxyde métallique, à l'interface décane-eau

L'objectif principal de ce test est d'étudier l'effet de la présence de différentes nanoparticules (TiO2 et ZnO) sur les propriétés rhéologiques de la couche d'adsorption de la lignine à l'interface décane-eau.

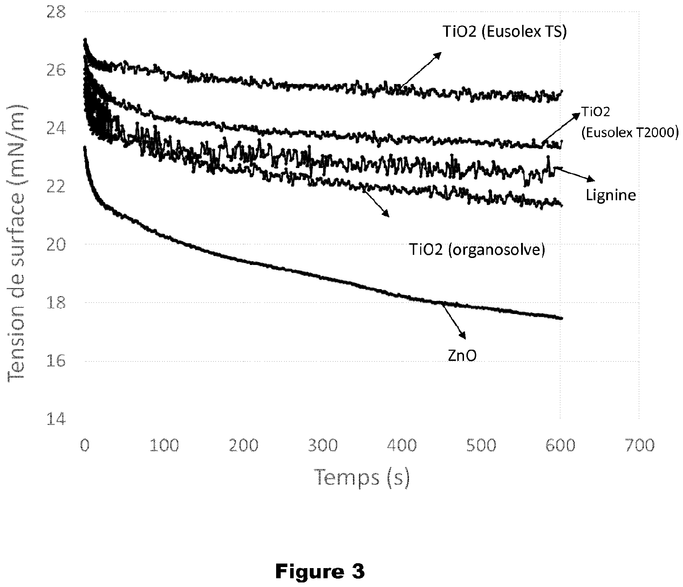

Sur la figure 3 sont présentées les courbes de cinétique d'adsorption de la lignine à l'interface décane-eau avec et sans nanoparticule.

D'après les résultats obtenus on constate qu'en présence de ZnO, la tension de surface décane-eau est le plus basse, tandis qu'avec TiO2 (Eusolex TS) et TiO2 (Eusolex T2000), les propriétés de surface de la lignine sont diminuées. Les TiO2 W877, au contraire, favorisent légèrement les propriétés de surface de la lignine.

L'effet de la présence des nanoparticules de ZnO et TiO2 sur les propriétés physico-chimiques de la couche d'adsorption de la lignine à l'interface décane/eau ont été rassemblées dans le tableau 4 suivant :

**Tableau 4**

| | Tension de surface (γ) (mN/m) | Pression de surface (π) (mN/m) | Module de dilatation (ε) (mN/m) |
|---|---|---|---|
| Interface eau/décane | 51.44 | 0 | 1.19 |
| Ech-1 (lignine seule) | 22.35 | 29.09 | 15.95 |
| Ech-2 (lignine + NPs-1) | 17.45 | 33.99 | 50.23 |
| Ech-3 (lignine + NPs-2 | 21.38 | 30.06 | 17.96 |
| Ech-4 (lignine + NPs-3) | 25.01 | 26.43 | 10.17 |
| Ech-5 (lignine + NPs-4) | 23.30 | 28.14 | 14.04 |

Donc, on voit clairement que les nanoparticules de ZnO augmentent le module de dilatation de la couche d'adsorption de la lignine.

### Exemple 5. Mesure des capacités anti-UV (test SPF) des émulsions décane/eau stabilisée avec différentes concentrations de lignine et des ratios lignine/particules TiO₂ variables

Différents échantillons ont été testés. L'échantillon témoin est une formulation hors invention, comprenant 2% de nanoparticules de TiO₂, permettant une comparaison des propriétés des émulsions 1, 2 et 3 selon l'invention. La densité de décane est 0.726 g/cm3 à 25°C, ce qui permet de calculer son pourcentage en poids dans l'émulsion.

**Tableau 5**

| **Echantillon** | **Eau** | | **Décane** | | | **Lignine** | | **TiO2** | | **Ratio nanoparticules / lignine** |
|---|---|---|---|---|---|---|---|---|---|---|
| | **ml (g)** | **%** | **ml** | **g** | **%** | **g** | **%** | **g** | **%** | |
| témoin | 3.5 | 41.22 | 6.5 | 4.719 | 55.58 | 0.1019 | 1.2 | 0.1698 | 2 | 1.66 |
| 1 | 3.5 | 41.82 | 6.5 | 4.719 | 56.38 | 0.1004 | 1.2 | 0.0502 | 0.6 | 0.5 |
| 2 ∗ | 3.5 | 41.95 | 6.5 | 4.719 | 56.55 | 0.1001 | 1.2 | 0.0250 | 0.3 | 0.25 |
| 3 ∗ | 3.5 | 42.02 | 6.5 | 4.719 | 56.66 | 0.0999 | 1.2 | 0.0099 | 0.12 | 0.1 |

| | | | | | | | | | | |
|---|---|---|---|---|---|---|---|---|---|---|
| ∗: pas selon l'invention | | | | | | | | | | |

Les mesures de facteur de protection solaire (SPF) et du facteur de protection UVA sont réalisées selon des techniques classiques bien connues de l'Homme du métier.

**Tableau 6**

| **Echantillon** | SPF±DS | FP-UVA ± DS | Ratio SPF/FP-UVA |
|---|---|---|---|
| **témoin** | 8,09 ± 0,68 | 6,03 ± 0,45 | 1,34 |
| **1** | 4,82 ± 0,66 | 3,98 ± 0,46 | 1,21 |
| **2** | 4,87 ± 0,37 | 3,82 ± 0,24 | 1,27 |
| **3** | 3,44 ± 0,22 | 2,90 ± 0,15 | 1,19 |

Il apparait que le meilleur SPF est obtenu lorsque la concentration de TiO₂ est la plus importante (2%) dans l'échantillon témoin.

Néanmoins, et de manière surprenante, on observe que la diminution du taux de TiO2 ne s'accompagne pas d'une diminution du même ordre de la protection contre les UVs ; en effet :
- l'échantillon 1 comprend 0.6% de TiO2 soit 3.3 fois moins que dans l'échantillon témoin: néanmoins, le SPF est diminué de 1.678 fois seulement, et le FP-UVA de 1.515 fois ;
- l'échantillon 3 comprend de très faible dose de TiO2 : 16.66 fois moins que dans l'échantillon témoin ; néanmoins, le SPF est diminué de seulement 2.35 fois et le FP-UVA de 2.079 fois.

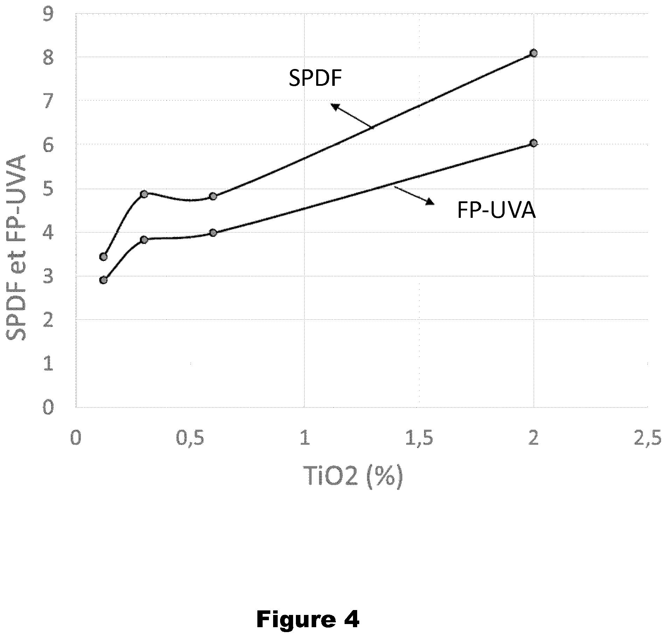

La figure 4 illustre cette augmentation modérée du SPF et du FP-UVA en fonction de la concentration en nanoparticules de TiO2.

Ainsi, l'association de nanoparticules de dioxyde de titane avec de la lignine permet de limiter la quantité de nanoparticules utilisée, tout en maintenant un facteur de protection solaire (SPF) et un facteur de protection contre les UVA suffisants pour la protection des composés actifs lipophiles.

### REFERENCES BIBLIOGRAPHIQUES

EP 518772
EP 518773
WO 2012/156652
US 2002/0182155
C. Pouteau, P. Dole, B. Cathala, L. Averous, N. Boquillon. Antioxidant Properties of Lignin in Polypropylene, Polymer Degradation and Stability ; 81 (2003) 9-18.
Rojas Orlando.; Bullon Johnny; Ysambertt Fredy; Forgiarini Ana; Salager Jean-L.; Argyropoulos Dimitris. Lignins as emulsion stabilizers. ACS symposium series, 2007, vol. 954, pp. 182-199.
H Shi, R Magaye, V Castranova, J Zhao: Titanium dioxide nanoparticles: a review of current toxicological data. Particle and Fibre Toxicology 2013 10:15
Rapport adopté par la Commission de cosmétologie du 15 mars 2011 (AFSSAPS). *Etat des connaissances relatif aux nanoparticules de dioxyde de titane et d'oxyde de zinc dans les produits cosmétiques en termes de pénétration cutanée, de génotoxicité et de cancérogenèse.* Saisine 2008 BCT0001
B. Monties. "Preparation of dioxane lignin fractions by acidolysis." Methods in Enzymology 161 (1988): 31-35.

## Revendications

1. Emulsion de type « huile-dans-eau » comprenant une phase aqueuse, une phase grasse, et une couche d'adsorption à l'interface des deux phases, ladite couche d'adsorption étant constituée d'une matrice de lignine dans laquelle sont incorporées des nanoparticules d'oxyde métallique,
**caractérisée en ce que** la lignine est présente en une quantité de 0.5% à 2% en poids, et que les nanoparticules d'oxyde métallique sont présentes en une quantité de 0.5% à 1.5% en poids, par rapport au poids total de l'émulsion.

2. Emulsion selon la revendication 1, **caractérisée en ce que** les nanoparticules d'oxyde métallique sont des particules de monoxyde de zinc ou des particules de dioxyde de titane.

3. Emulsion selon la revendication 1 ou 2, **caractérisée en ce que** les nanoparticules d'oxyde métallique sont liées à la matrice de lignine par des liaisons non-covalentes.

4. Emulsion selon l'une des revendications 1 à 3, **caractérisée en ce que** les nanoparticules d'oxyde métallique sont incorporées à la matrice de lignine par sonication.

5. Emulsion selon la revendication 1 ou 2, **caractérisée en ce que** les nanoparticules d'oxyde métallique sont liées à la matrice de lignine par des liaisons covalentes.

6. Emulsion selon l'une des revendications 1 à 5, **caractérisée en ce que** les nanoparticules d'oxyde métallique sont présentes en un ratio en poids nanoparticules / lignine compris entre 0.1 et 0.8, et plus préférentiellement sont présentes en un ratio en poids nanoparticules / lignine d'environ 0.5.

7. Emulsion selon l'une des revendications 1 à 6, **caractérisée en ce que** la lignine est d'origine naturelle.

8. Emulsion selon la revendication 7, **caractérisée en ce que** la lignine est issue d'un procédé d'extraction de cellulose à partir de plantes.

9. Emulsion selon l'une des revendications 1 à 6, **caractérisée en ce que** la lignine est d'origine synthétique.

10. Emulsion selon l'une des revendications 1 à 9, **caractérisée en ce que** la phase grasse comprend un solvant organique choisi parmi le cyclopentane, l'hexane, le méthylcyclohexane, l'heptane, le décane, le dodécane et le toluène.

## Patentansprüche

1. Emulsion vom Typ "Öl-in-Wasser", umfassend eine wässrige Phase, eine fette Phase und eine Adsorptionsschicht an der Schnittstelle der zwei Phasen, wobei die Adsorptionsschicht aus einer Matrix aus Lignin besteht, in der Nanopartikel aus Metalloxid eingeschlossen sind,
**dadurch gekennzeichnet, dass** das Lignin in einer Menge von 0,5 Gew% bis 2 Gew% vorhanden ist, und dass die Nanopartikel aus Metalloxid in einer Menge von 0,5 Gew% bis 1,5 Gew% mit Bezug auf das Gesamtgewicht der Emulsion vorhanden sind.

2. Emulsion nach Anspruch 1, **dadurch gekennzeichnet, dass** die Nanopartikel aus Metalloxid Partikel aus Zinkmonoxid oder Partikel aus Titandioxid sind.

3. Emulsion nach Anspruch 1 oder 2, **dadurch gekennzeichnet, dass** die Nanopartikel aus Metalloxid mit der Matrix aus Lignin durch nicht kovalente Bindungen verbunden sind.

4. Emulsion nach einem der Ansprüche 1 bis 3, **dadurch gekennzeichnet, dass** die Nanopartikel aus Metalloxid in die Matrix aus Lignin durch Ultraschallbehandlung eingeschlossen sind.

5. Emulsion nach Anspruch 1 oder 2, **dadurch gekennzeichnet, dass** die Nanopartikel aus Metalloxid mit der Matrix aus Lignin durch kovalente Bindungen verbunden sind.

6. Emulsion nach einem der Ansprüche 1 bis 5, **dadurch gekennzeichnet, dass** die Nanopartikel aus Metalloxid in einem Gewichtsverhältnis Nanopartikel-Lignin vorhanden sind, das zwischen 0,1 und 0,8 liegt, und insbesondere in einem Gewichtsverhältnis Nanopartikel-Lignin von ungefähr 0,5 vorhanden sind.

7. Emulsion nach einem der Ansprüche 1 bis 6, **dadurch gekennzeichnet, dass** das Lignin natürlichen Ursprungs ist.

8. Emulsion nach Anspruch 7, **dadurch gekennzeichnet, dass** das Lignin aus einem Verfahren zur Extraktion von Cellulose aus Pflanzen stammt.

9. Emulsion nach einem der Ansprüche 1 bis 6, **dadurch gekennzeichnet, dass** das Lignin synthetischen Ursprungs ist.

10. Emulsion nach einem der Ansprüche 1 bis 9, **dadurch gekennzeichnet, dass** die fette Phase ein organisches Lösemittel umfasst, ausgewählt aus Cyclopentan, Hexan, Methylcyclohexan, Heptan, Decan, Dodecan und Toluen.

## Claims

1. "Oil-in-water" type emulsion comprising an aqueous phase, a fatty phase, and an adsorption layer at the interface of the two phases, wherein the adsorption layer consists of a lignin matrix in which are incorporated metal oxide nanoparticles,
**characterized in that** the lignin is present in an amount of 0.5% to 2% by weight, and that the metal oxide nanoparticles are present in an amount of 0.5% to 1.5% by weight, relative to the total weight of the emulsion.

2. Emulsion according to claim 1, **characterized in that** the metal oxide nanoparticles are zinc oxide particles or titanium dioxide particles.

3. Emulsion according to claim 1 or 2, **characterized in that** the metal oxide nanoparticles are bonded to the lignin matrix by non-covalent bonds.

4. Emulsion according to one of the claims 1 to 3, **characterized in that** the metal oxide nanoparticles are incorporated into the lignin matrix by sonication.

5. Emulsion according to claim 1 or 2, **characterized in that** the metal oxide nanoparticles are bonded to the lignin matrix by covalent bonds.

6. Emulsion according to one of the claims 1 to 5, **characterized in that** the metal oxide nanoparticles are present in a weight ratio of nanoparticles/lignin between 0.1 and 0.8, and more preferably in a weight ratio of nanoparticles/lignin of about 0.5.

7. Emulsion according to one of the claims 1 to 6, **characterized in that** the lignin is of natural origin.

8. Emulsion according to claim 7, **characterized in that** the lignin is derived from a method for extracting cellulose from plants.

9. Emulsion according to one of the claims 1 to 6, **characterized in that** the lignin is of synthetic origin.

10. Emulsion according to one of the claims 1 to 9, **characterized in that** the fatty phase comprises an organic solvent selected from among cyclopentane, hexane, methylcyclohexane, heptane, decane, dodecane and toluene.
